# EUROPÄISCHE PATENTANMELDUNG

(11) **EP 2 082 727 A2**
(43) Veröffentlichungstag der Anmeldung: **29.07.2009**
(21) Anmeldenummer: 08170433.0
(22) Anmeldetag: 02.12.2008
(51) Int. Cl.: A61K 8/81, A61Q 5/06

(54) **Haarspray mit besonders hohem Langzeithalt**

(30) Priorität: 25.01.2008 DE 102008006227
(71) Anmelder: Beiersdorf AG, 20253 Hamburg (DE)
(72) Erfinder: Saß, Viola, 25488 Holm (DE); Dingler, Christian, 22527 Hamburg (DE); Heuer, Björn, 22145 Hamburg (DE)

(57) **Zusammenfassung**

Haarbehandlungsmittel enthaltend ein kationisch amphoteres, filmbildendes Polymer, aufgebaut aus den Monomeren Vinylpyrrolidon, *tert-*Butylacrylat, Methacrylsäure, Dimethylaminopropylmethacrylamid und Methacryloylaminopropyldimethylethyl Ammonium Ethylsulfat, mit den Vorgaben, dass der Anteil Methacrylsäure geringer ist als der Anteil Dimethylaminopropylmethacrylamid und dass das Polymer mit einer geeigneten Säure neutralisiert ist, sowie mindestens ein Additiv zur Verbesserung des Trocknungsverhaltens des Polymerfilms.

## Beschreibung

Haarsprays sind Haarstyling-Produkte, die typischer Weise in eine fertig erstellte Frisur gesprüht werden, um den gewünschten Look zu fixieren. Dabei finden bevorzugt Aerosol-Sprays Verwendung, die mit Hilfe eines Flüssig-Gases als Treibmittel versprüht werden, Pump-Haarsprays ohne Treibmittel sind aufgrund des schlechteren Sprühbildes weniger verbreitet.

Haarsprays bestehen aus einer alkoholischen, bzw. wässrig-alkoholischen Lösung eines oder mehrerer fixierender Polymere, die zusammen mit dem Treibgas (Gase, die ab einem bestimmten Druck, in der Regel zwischen 2 und 5 bar flüssig vorliegen, z. B. Dimethylether, Propan/Butan-Mischungen oder Fluorkohlenwasserstoffe) in einer druckdichten Aerosol-Dose aus Weißblech oder Aluminium abgefüllt sind. Versprüht wird das Haarspray einen Sprühkopf, der bei Betätigung ein Ventil, welches mit einem Steigrohr versehen ist, öffnet und somit den Austrag des Produktes ermöglicht.

Das Sprühbild eines Haarsprays wird im wesentlichen über die Zusammensetzung der Polymerlösung, die Art und die Menge des verwendeten Treibmittels sowie die Beschaffenheit von Ventil und Düse beeinflusst.

Im Falle der Polymerlösung sind die Lösungsviskosität, sowie die Anteile an Wasser und Alkohol für das Sprühbild verantwortlich. Eine hohe Lösungsviskosität und ein hoher Wasseranteil führen in der Regel zu größeren Aerosol-Tröpfchen, die nach dem Sprühen auf das Haar langsamer trocknen und ein starkes Verkleben verursachen. Die Lösungsviskosität ist von der Art und Konzentration des verwendeten fixierenden Polymers abhängig. Als Polymere in Haarsprays finden hauptsächlich statistische Copolymere auf Basis von (Meth)Acrylsäure Verwendung.

Neben dem Einfluss der Zusammensetzung der Wirkstofflösung (Polymer, Wasser und Alkohol), spielt das Treibmittel eine entscheidende Rolle für das Sprühbild. In Europa werden praktisch ausnahmslos Dimethyleter und Mischungen aus Propan, Isobutan und Butan eingesetzt. Bei Verwendung von Dimethylether entscheiden Konzentration und Wassergehalt den Druck in der Aerosoldose. Bei 25°C können etwa zwischen 3,5 und 5,5 bar eingestellt werden. Ein hoher Wasseranteil sorgt hierbei für eine schlechtere Löslichkeit des Dimethylethers in der Wirkstofflösung und somit zu einem erhöhten Druck in der Aerosoldose. Eine erhöhte Konzentration von Dimethylether sorgt ebenfalls für einen erhöhten Druck, da die Lösung im Wesentlichen mit Dimethylether bereits gesättigt ist, befindet sich zusätzliches Treibmittel dann hauptsächlich in der Gasphase. Mit einer Mischung aus Kohlenwasserstoffen lässt sich der Druck über die Anteile Propan, Isobutan und Butan einstellen. Generell ist die Löslichkeit der Kohlenwasserstoffe in der Haarspray-Wirkstofflösung deutlich schlechter als die von Dimethether, weswegen Kohlenwasserstoff-Haarsprays in der Regel mit deutlich weniger Treibmittel formuliert werden müssen. Mit erhöhtem Druck werden in der Regel auch die Tröpfchen im Aerosolnebel kleiner.

Eine letzte Möglichkeit der Beeinflussung des Sprühbildes liegt in der Wahl von Ventil und Düse. Mit der Kegelöffnung des Ventils wird in der Regel die Sprührate eingestellt. Zudem sorgt eine kleine Kegelöffnung zusätzlich für kleinere Tröpfchen im Aerosolnebel. Die Verwirbelung des Sprühstrahls wird schließlich durch die Wahl der Düse beeinflusst.

Der Verbraucher erwartet von einem Haarspray verschiedene Dinge. Zum ersten muss das Produkt der Frisur zuverlässigen Halt verleihen, möglichst den ganzen Tag auch bei Wind, Regen, Brührungen usw. Des Weiteren soll die Frisur aber natürlich aussehen, sich auch natürlich anfühlen und das Produkt am Abend ohne Probleme auskämmbar sein. Eine hohe, und damit zuverlässige Fixierung wird aber nur durch starke Verknüpfungspunkte zwischen einzelnen Haaren erreicht. Hierzu müssen die im Haar verbleibenden Polymertröpfchen möglichst groß sein, was entweder durch eine hohe Polymerkonzentration oder große Tröpfchen im Aerosolnebel realisiert werden kann. Da solche starken Verknüpfungspunkte aber immer auch zu einem unnatürlichen Look, einem unnatürlichen Haargefühl und zu besonders schwerem Ausbürsten am Abend führen, stellen solche Produkte die Verbraucher selten zufrieden.

Eine Aufgabe der vorliegenden Erfindung war es nun, ein Haarspray zu finden, welches bei möglichst geringer Einsatzkonzentration an fixierendem Polymer, und damit maximaler Natürlichkeit des Looks, einen zuverlässigen Halt den ganzen Tag liefert, dabei einen angenehmen Griff aufweist und gutes Trocknungsverhalten zeigt.

Die WO 2001/062809 (BASF) Kosmetika mit Copolymeren aus drei bestimmten Comonomeren.

Die WO 2005/058988 offenbart bestimmte ampholytische Copolymere die sich zu Festigungszwecken eignen sollen.

Die WO 2004/058837 offenbart bestimmte ampholytische Copolymere, kosmetische oder pharmazeutische Mittel diese enthaltend in einer Kombination mit einer Vielzahl weiterer Komponenten in verschiedenen Produktformen, darunter auch Haarfestiger.

Die WO 1995/035087 offenbart ein haarfixierendes amphotheres Polymer, das Hydroxyethylmethacrylat als Comonomer enthalten kann.

Die US 4237253 offenbar bestimmte Copolymere auf Acrylbasis und deren Herstellverfahren.

Die EP 0 330 174 offenbart Haarfixiergele mit vernetzten Carboxyvinyl-Polymeren, amphotheren Polymeren und 85 bis 99.3 % wässriges und/oder alkoholisches Lösungsmittel.

Es mangelte dem Stand der Technik an Zubereitungen, die die Nachteile des Stand der Technik nicht aufwiesen.

Es hat sich nun für den Fachmann überraschend gezeigt, dass die Verwendung eines kationisch amphoteren Polymers, aufgebaut aus den Monomeren Vinylpyrrolidon, *tert*-Butylacrylat, Methacrylsäure, Dimethylaminopropylmethacrylamid und Methacryloylaminopropyldimethylethyl Ammonium Ethylsulfat, mit den Vorgaben, dass der Anteil Methacrylsäure geringer ist als der Anteil Dimethylaminopropylmethacrylamid und dass das Polymer mit einer geeigneten Säure neutralisiert ist, in Kombination mit geeigneten Additiven, oder bei Verwendung von Propan/Butan Mischungen als Treibmittel den Nachteilen des Standes der Technik abhilft.

Die Erfindung umfasst insbesondere die Gegenstände des Patentanspruch 1 und die der restlichen Patentansprüche.

Der resultierende Polymerfilm liefert deutlich bessere mechanische Eigenschaften in Bezug auf maximale Biegespannung, maximale Biegung und maximale Arbeit zum Brechen des Films. Aufgrund dieser hohen mechanischen Belastbarkeit hält die Frisur auch bei Belastung deutlich besser als mit anderen Haarsprays. Zudem sorgen die Additive, bzw. die Verwendung von Propan/Butan Mischungen dafür, dass das Trocknungsverhalten des Sprays (Klebrigkeit bzw. Trocknungszeit) besser ist als bei Verwendung von Sprays gemäß Stand der Technik.

Es ist von Vorteil, wenn zusätzlich Ammomium Hydroxid und MEA/MIPA Borat als Additiv zur Verhinderung der Dosenkorrosion eingesetzt wird.

Es ist von Vorteil, wenn Dimethicone (z. B. Mirasil DM 100; Rhodia) oder Cyclopentasiloxan (z. B. Dow Corning 245) als lineares oder cyclisches Silikon eingesetzt werden.

Es ist von Vorteil, wenn PEG-12 Dimethicone (z. B. Dow Corning 193 Fluid) als PEG- und/oder PPG-modifiziertes Silikon eingesetzt wird.

Es ist von Vorteil, wenn Lanolin-Öl, Jojoba-Öl, Avocado-Öl, Oliven-Öl, Sonnenblumen-Öl, Weizenkeim-Öl oder Paraffinum Liquidum als natürliches oder synthetisches Öl eingesetzt werden.

Es ist von Vorteil, wenn Brennnessel-, Süßholz-, Ginko-, Aloe Vera-, Seerosen-, Bambus-, Granatapfel-, Sonnenblumen-, Weizenkeim-, Kamillen- oder Ginsengextrakt als Pflanzenextrakt eingesetzt werden.

Es ist von Vorteil, wenn Glycerin oder Propylenglycol als mehrwertiger Alkohol eingesetzt werden.

Es ist von Vorteil wenn Ubichinon als (Co-)Enzym eingesetzt wird.

Es ist von Vorteil, wenn Niacinamid (B3) und/ oder Panthenol (ProVitB5) als Vitamine eingesetzt werden.

Es ist von Vorteil, wenn zusätzlich 4-Benzophenon als UV-Filter eingesetzt wird.

Es ist von Vorteil, wenn zusätzlich Oryzanol als Antioxidationsmittel eingesetzt wird.

Es ist von Vorteil, wenn zusätzlich Algen-, Caviar-, oder Honigextrakt als Extrakte eingesetzt werden.

Es ist von Vorteil, wenn zusätzlich Seiden-, Weizen- oder Sojaproteine als Proteine eingesetzt werden.

Es ist von Vorteil, wenn zusätzlich Seiden-, Weizen- oder Sojaproteinhydrolysate als Proteinhydrolysate eingesetzt werden.

Es ist von Vorteil, wenn zusätzlich farbige Pigmente eingesetzt werden.

Alle Prozentangaben in dieser Schrift sind als Massenprozent zu verstehen, sofern nicht anders gekennzeichnet.

### Beispiele

### Verwendete Methoden

### Halt:

Zyklischer 3-Punkt Biegeversuch. Die Haarsträhne (20 cm, 2 g Gewicht) wird mit 0,2 ml Wirkstofflösung (ohne Treibmittel) behandelt und bei 25°C und 55 % r.F. für mindestens 24 Stunden konditioniert.

Anschließend wird die Strähne in einem 3-Punkt Biegeversuch wiederholt um eine gleichmäßig zunehmende Strecke (jeweils 0,1 mm mehr) gedehnt und die dafür benötigte Kraft gemessen.

Die Stärke des Halts wird über die maximale Biegespannung und die insgesamt verrichtete Arbeit (Kraft/Weg Integral) beschrieben.

Die Elastizität wird über die Auslenkung beschrieben, bei der die maximale Kraft auftritt.

### Klebrigkeit:

### Texture Analyzer TA.XTplus (Stable Micro Systems)

Adhesive Test, test speed 0,1 mm/s, force 200 g, time 10 s, distrance 2 mm, trigger type automatic 5 g.

Das Aerosol wird für 3,0 s aus einer Entfernung von 15 cm auf eine Plexiglasplatte gesprüht, auf welche Papier geklebt ist, das auf der Rückseite eine Silikonbeschichtung aufweist.

Die Messung der Klebrigkeit erfolgt mit einem Edelstahlzylinder (Durchmesser 0,25 Zoll) jede Minute, bis keine Kraft mehr messbar ist. Als Klebrigkeit wird die maximale Kraft als Mittelwert aus mindestens 5 Messungen angegeben. Als Trockenzeit wird die Zeit definiert, bei der der Mittelwert der Kraft aus allen Messungen kleiner als 0,5 N ist.

Polymer 1: statistisches, lineares Copolymer aus Vinylpyrrolidon, *tert*-Butylacrylat, Methacrylsäure, Dimethylaminopropylmethacrylamid und Methacryloylaminopropyldimethylethyl Ammonium Ethylsulfat, neutralisiert mit Phosphorsäure auf pH 6,0.

### Beispiel 1 (nicht erfindungsgemäß):

| | |
|---|---|
| Octylacrylamide/Acrylates/Butylaminoethyl | 3 % |
| Methacrylate Copolymer (Amphomer 28- | |
| 4910 von National Starch) | |
| AMP-95 | 0,7 % |
| Wasser | 8 % |
| Ethanol | 36 % |
| Dimethylether | 50 % |
| PEG-12 Dimethicon | 1 % |

| | |
|---|---|
| Maximale Biegekraft: 3000 mN Auslenkung bei maximaler Biegekraft: 7,1 mm Klebrigkeit: 3,7 N Trockenzeit: 10 min | |

### Beispiel 2 (nicht erfindungsgemäß):

| | |
|---|---|
| Octylacrylamide/Acrylates/Butylaminoethyl | 3 % |
| Methacrylate Copolymer (Amphomer 28- | |
| 4910 von National Starch) | |
| AMP-95 | 0,7 % |
| Ethanol | 61,3 % |
| Propan / Butan (2,7 bar) | 35 % |

| | |
|---|---|
| Maximale Biegekraft: 3100 mN Auslenkung bei maximaler Biegekraft: 6,5 mm Klebrigkeit: 3,3 N Trockenzeit: 9 min | |

### Beispiel 3 (nicht erfindungsgemäß):

| | |
|---|---|
| Polymer 1 | 3 % |
| Wasser | 41,7 % |
| Ethanol | 15 % |
| Dimethylether | 40 % |

| | |
|---|---|
| Maximale Biegekraft: 4300 mN Auslenkung bei maximaler Biegekraft: 11,2 mm Klebrigkeit: 3,7 N Trockenzeit: 58 min | |

### Beispiel 4 (nicht erfindungsgemäß):

| | |
|---|---|
| Polymer 1 | 3 % |
| Wasser | 12,5 % |
| Ethanol | 34,5 % |
| Dimethylether | 50 % |

| | |
|---|---|
| Maximale Biegekraft: 4300 mN Auslenkung bei maximaler Biegekraft: 11,2 mm Klebrigkeit: 5,5 N Trockenzeit: 13 min | |

### Beispiel 5 (erfindungsgemäß):

| | |
|---|---|
| Polymer 1 | 3 % |
| Ethanol | 62 % |
| Propan / Butan (2,7 bar) | 35 % |

| | |
|---|---|
| Maximale Biegekraft: 4300 mN Auslenkung bei maximaler Biegekraft: 11,2 mm Klebrigkeit: 2,1 N Trockenzeit: 8 min | |

### Beispiel 6 (erfindungsgemäß):

| | |
|---|---|
| Polymer 1 | 3 % |
| Wasser | 12,5 % |
| Ethanol | 33,5 % |
| Dimethylether | 50 % |
| Panthenol | 1 % |

| | |
|---|---|
| Maximale Biegekraft: 4300 mN Auslenkung bei maximaler Biegekraft: 11,2 mm Klebrigkeit: 4,5 N Trockenzeit: 12 min | |

### Beispiel 7 (erfindungsgemäß):

| | |
|---|---|
| Polymer 1 | 3 % |
| Wasser | 12,5 % |
| Ethanol | 33,5 % |
| Dimethylether | 50 % |
| Glycerin | 1 % |

| | |
|---|---|
| Maximale Biegekraft: 4250 mN Auslenkung bei maximaler Biegekraft: 11,2 mm Klebrigkeit: 4,4 N Trockenzeit: 13 min | |

### Beispiel 8 (erfindungsgemäß):

| | |
|---|---|
| Polymer 1 | 3 % |
| Wasser | 12,5 % |
| Ethanol | 33,5 % |
| Dimethylether | 50 % |
| PEG/PPG-17/18 Dimethicon | 1 % |
| Maximale Biegekraft: 4250 mN Auslenkung bei maximaler Biegekraft: 11,2 mm Klebrigkeit: 4,4 N Trockenzeit: 13 min | |

Die erfindungsgemäßen Beispiele zeigen als einzige sowohl sehr gute Halteleistungen als auch im Vergleich zu Formeln aus dem Stand der Technik reduzierte Klebrigkeiten (mind. -20 %) und/oder Trocknungszeiten.

## Patentansprüche

1. Haarbehandlungsmittel enthaltend ein kationisch amphoteres, filmbildendes Polymer, enthaltend die Monomere Vinylpyrrolidon, *tert*-Butylacrylat, Methacrylsäure, Dimethylaminopropylmethacrylamid und Methacryloylaminopropyldimethylethyl Ammonium Ethylsulfat, mit den Vorgaben, dass der Anteil Methacrylsäure geringer ist als der Anteil Dimethylaminopropylmethacrylamid und dass das Polymer mit einer geeigneten Säure neutralisiert ist, sowie mindestens ein Additiv zur Verbesserung des Trocknungsverhaltens des Polymerfilms.

2. Haarbehandlungsmittel enthaltend ein kationisch amphoteres, filmbildendes Polymer, enthaltend die Monomere Vinylpyrrolidon, *tert*-Butylacrylat, Methacrylsäure, Dimethylaminopropylmethacrylamid und Methacryloylaminopropyldimethylethyl Ammonium Ethylsulfat, mit den Vorgaben, dass der Anteil Methacrylsäure geringer ist als der Anteil Dimethylaminopropylmethacrylamid und dass das Polymer mit einer geeigneten Säure neutralisiert ist, sowie Propan und Butan.

3. Mittel nach Patentanspruch 1 oder 2 **dadurch gekennzeichnet, dass** der Anteil saurer Monomere geringer ist als der Anteil basischer Monomere.

4. Mittel nach einem der vorangehenden Patentansprüche **dadurch gekennzeichnet, dass** das filmbildende Polymer in einer Konzentration zwischen 0,5 und 7,5 % eingesetzt wird.

5. Mittel nach einem der vorangehenden Patentansprüche **dadurch gekennzeichnet, dass** das Treibmittel in einer Konzentration zwischen 20 und 70 % eingesetzt wird.

6. Mittel nach einem der vorangehenden Patentansprüche **dadurch gekennzeichnet, dass** das Additiv zur Verbesserung des Trocknungsverhaltens des Polymerfilms ausgewählt ist unter linearen oder cyclischen Silikonen, PEG- und/oder PPG-modifizierten Silikonen, natürlichen oder synthetischen Ölen, Planzenextrakten, mehrwertigen Alkoholen, Vitaminen oder anderen Naturstoffen.

7. Mittel nach einem der vorangehenden Patentansprüche **dadurch gekennzeichnet, dass** zusätzlich UV-Filter, Antioxidationsmittel, Silikone, Öle, Extrakte, Proteine und/oder Proteinhydrolysate, Enzyme, Co-Enzyme, Pigmente und/oder Parfum enthalten sind.

8. Mittel nach einem der vorangehenden Patentansprüche **dadurch gekennzeichnet, dass** das Additiv zur Verbesserung des Trocknungsverhaltens des Polymerfilms in einer Konzentration zwischen 0,01 und 5 % eingesetzt wird.

9. Mittel nach einem der vorangehenden Patentansprüche **dadurch gekennzeichnet, dass** zusätzlich ein Additiv zur Verhinderung der Dosenkorrosion eingesetzt wird, besonders bevorzugt in einer Konzentration zwischen 0,05 und 2,5 %.

10. Mittel nach einem der vorangehenden Patentansprüche **dadurch gekennzeichnet, dass** zusätzlich mindestens ein weiteres anionisches, kationisches, nicht ionisches oder amphoteres filmbildendes Polymer in einer Konzentration von 0,1 bis 7,5% eingesetzt wird.

11. Mittel nach einem der vorangehenden Patentansprüche **dadurch gekennzeichnet, dass** der pH-Wert der wässrigen Zubereitung zwischen 5 und 7, besonders bevorzugt zwischen 5,5 und 6,5, ganz besonders bevorzugt zwischen 5,7 und 6,3 liegt.

12. Mittel nach einem der vorangehenden Patentansprüche **dadurch gekennzeichnet, dass** die Monomeranteile im kationischen amphoteren, filmbildenden Polymer für Vinylpyrrolidon 20 % bis 30 %, besonders bevorzugt 25 %, für *tert*-Butylacrylat 40 % bis 50 %, besonders bevorzugt 47 %, für Methacrylsäure 2 % bis 5 %, besonders bevorzugt 3 %, für Dimethylaminopropylmethacrylamid 5 % bis 15 %, besonders bevorzugt 10 % und für Methacryloylaminopropyldimethylethyl Ammonium Ethylsulfat 10 % bis 20 %, besonders bevorzugt 15 % betragen.

13. Verwendung eines kationischen amphoteren, filmbildenden Polymers nach einem der vorangehenden Patentansprüche zur Verbesserung der Halteleistungen einer kosmetischen haarfixierenden Zubereitung, zur Verringerung ihrer Klebrigkeit und/oder der Trocknungszeiten des auf das Haar aufgesprühten Produkts.
